# EUROPÄISCHE PATENTANMELDUNG

(11) **EP 1 063 265 A1**
(43) Veröffentlichungstag der Anmeldung: **27.12.2000**
(21) Anmeldenummer: 00111893.4
(22) Anmeldetag: 13.06.2000
(51) Int. Cl.: C09C 1/30, C09C 1/00, A61K 7/42, A61K 7/027, A61K 7/031, A61K 7/043, C09D 7/12

(54) **Anorganische sphärische Absorptionspigmente**

(30) Priorität: 24.06.1999 DE 19929109
(71) Anmelder: MERCK PATENT GmbH, 64293 Darmstadt (DE)
(72) Erfinder: Anselmann, Ralf, Dr., 67305 Ramsen (DE)

(57) **Zusammenfassung**

Anorganische sphärische Absorptionspigmente, die aus Siliciumdioxid-Kugeln mit einem Durchmesser von 50 nm bis 50 µm und einer Beschichtung aus einem Metalloxid mit Ausnahme von Titandioxid oder einem Metalloxid und einem farbgebenden Material bestehen. Das Metalloxid ist Fe₂O₃, FeO(OH), Fe₃O₄ oder Cr₂O₃. Das farbgebende Material ist Berliner Blau, Carminrot, ein organisches Pigment oder ein organischer Farbstoff.

## Beschreibung

Die Erfindung betrifft anorganische Absorptionspigmente, die aus kugelförmigem Siliciumdioxid als Kern und einer Beschichtung aus Metalloxiden bestehen.

Anorganische Absorptionspigmente müssen vor ihrer Anwendung in zu pigmentierenden System in eine Form gebracht werden, die eine leichte Dispergierung und eine reproduzierbare Farbe ermöglicht. Diese Vorbehandlungen der Pigmente, beispielsweise Mahlen, die entscheidend die Qualität des Endproduktes beeinflussen, sind zeitaufwendig und teuer. Nachteilig ist weiterhin, daß beim Benetzen die Farbe der Pigmente verändert wird. Für kosmetische Formulierungen müssen die Pigmente zusätzlich noch ein gutes Hautgefühl haben, das die klassischen anorganischen Absorptionspigmente nur in geringem Umfang besitzen.

Aus JP 62-288 662 ist ein gelbes Pigment bekannt, das aus einem Kern aus Siliciumdioxidpartikeln und darauf haftenden Goethit-Partikeln besteht. Goethit ist α-FeO(OH), das auch als Nadeleisenerz bezeichnet wird. Die Herstellung erfolgt durch Mahlen der beiden Komponenten in trockenen Zustand oder durch Vermischen in einer wäßrig-alkalischen Lösung und nachfolgendem Trocknen. Dieses Pigment hat den Nachteil, daß es im Anwendungssystem nicht stabil ist und sich teilweise in seine Komponenten auftrennt. Für kosmetische Anwendungen besitzt es kein ausreichend gutes Hautgefühl.

Außerdem ist die Farbe des Pigmentes schwach und wenig brillant.

Bereits bekannt ist die Beschichtung von kugelförmigem Siliciumdioxid im Naßverfahren mit Titandioxid. JP 06-011 872 beschreibt kugelförmiges Siliciumdioxid, das mit Titandioxid beschichtet ist. Das Pigment wird als Maskierungsmittel in kosmetischen Formulierungen eingesetzt.

WO 94/21 733 beschreibt ein Pigment mit hohem Lichtstreuungsvermögen, bestehend aus einem Siliciumdioxid-Kern, einer ersten Schicht aus Titandioxid und einer zweiten Schicht aus Siliciumdioxid, Aluminiumsilikat oder Aluminiumoxid.

Aufgabe der Erfindung ist es, Absorptionspigmente mit großer Farbreinheit und Homogenität bereitzustellen, die ohne zusätzliche Vorbehandlung in das zu pigmentierende System eingearbeitet werden können und nur geringe Farbänderungen beim Benetzen zeigen.

Diese Aufgabe wird gemäß der Erfindung gelöst durch anorganische sphärische Absorptionspigmente, bestehend aus Siliciumdioxid-Kugeln mit einem Durchmesser von 50 nm bis 50 µm und einer Beschichtung aus einem Metalloxid mit Ausnahme von Titandioxid und/oder einem farbgebenden Material.

Weiterhin wird diese Aufgabe gemäß der Erfindung gelöst durch ein Verfahren zur Herstellung der erfindungsgemäßen Pigmente, indem Siliciumdioxid-Kugeln mit einem Durchmesser von 50 nm bis 50 µm in deionisiertem Wasser bei einer Temperatur von 50 bis 90 °C in einer Konzentration von 1 bis 30 Gew.-% dispergiert werden, 5 bis 40 %ige Metallsalzlösungen bei einem pH-Wert von 1 bis 10 mit einer Geschwindigkeit von 0,0001 bis 0,1 mg Metallsalz pro Minute und pro m² der Siliciumdioxid-Kugeln zugesetzt werden, wobei der pH-Wert durch gleichzeitige Zugabe einer Base konstant gehalten wird, die beschichteten Siliciumdioxid-Kugeln abgetrennt, mit vollentsalztem Wasser oder organischen Lösemitteln gewaschen und bei 80 bis 150 °C getrocknet und bei 300 bis 1000 °C geglüht werden. In einer weiteren Ausführungsform des erfindungsgemäßen Verfahrens wird auf eine dünne Metalloxidschicht zusätzlich noch ein farbgebendes Material aufgefällt.

Gegenstand der Erfindung ist außerdem die Verwendung des erfindungsgemäßen Pigmentes für die Pigmentierung von Lacken, Druckfarben, Kunststoffen und Formulierungen der pflegenden und dekorativen Kosmetik.

Als Ausgangsprodukt für die Herstellung der Pigmente wird kugelförmiges Siliciumdioxid mit einem Durchmesser von 50 nm bis 50 µm eingesetzt. Bedingt durch das Herstellungsverfahren besitzen die Kugeln keinen einheitlichen Durchmesser sondern ein Verteilungsspektrum bezüglich ihrer Partikelgröße. Geeignet als Ausgangsprodukt sind Fraktionen, bei denen 99 % der Partikel kleiner als 50 µm sind. Bevorzugt werden Fraktionen, bei denen 90 % der Partikel kleiner als 20 µm sind.

Die Herstellung von kugelförmigem Siliciumdioxid ist bekannt. Sie erfolgt durch Hydrolyse organischer oder anorganischer Siliciumverbindungen in einem Emulsionsverfahren. Derartige Verfahren sind beispielsweise in DE 21 55 281, DE 26 10 852, GB 1 141 924 und EP 0 162 716 beschrieben. Kugelförmiges Siliciumdioxid ist kommerziell verfügbar. Unter der Bezeichnung Ronasphere® wird von Merck KGaA kugelförmiges Siliciumdioxid mit einer Partikelgröße von kleiner 20 µm angeboten.

Die Beschichtung der Siliciumdioxid-Kugeln mit Metalloxiden oder farbgebenden Materialien, wie Berliner Blau, Carminrot sowie organischen Pigmenten und Farbstoffen erfolgt nach bekannten Verfahren.

Bei der Beschichtung mit Eisen-(III)-oxid kann sowohl von Eisen(III)-salzen ausgegangen werden, wie es zum Beispiel in US 3 087 828 und US 3 087 829 beschrieben ist, als auch von Eisen(II)-salzen, wie in US 3 874 890 beschrieben, wobei der zunächst gebildete Überzug von Eisen(II)-hydroxid zum Eisen(III)-oxidhydrat oxidiert wird. Bevorzugt wird von Eisen(III)-salzen ausgegangen. Hierzu wird einer wäßrigen Suspension des kugelförmigen Siliciumdioxids bei einer Temperatur von 60 bis 90 °C und bei einem pH-Wert von 2,5 bis 4,5 eine Eisen(III)-chloridlösung zudosiert. Der pH-Wert wird durch gleichzeitige Dosierung von 32%iger Natronlauge konstant gehalten. Dieses Verfahren ist in DE 196 18 568 beschrieben.

Das bei 300 bis 900 °C kalzinierte Pigment besitzt eine orangene bis rote Farbe.

Bei der Beschichtung mit gelbem FeO(OH) in der Goethitmodifikation wird von einem Gemisch aus Eisen(III)sulfat und Eisen(II)sulfat ausgegangen, das bei einem pH-Wert von 2,5 bis 4,5 zu einer auf 60 bis 90 °C erhitzten Suspension der Siliciumdioxid-Kugeln zudosiert wird, wobei der pH-Wert durch gleichzeitige Zugabe von 32%iger Natronlauge konstant gehalten wird.

Die Beschichtung mit gelbem FeO(OH) in der Goethitmodifikation kann auch nach dem in EP 0 659 843 beschriebenen Verfahren erfolgen. Hierbei wird in einer Stickstoffatmosphäre eine wäßrige FeSO₄-Lösung mit einem pH-Wert von 1,5 in eine auf 70 °C erhitzte Suspension von kugelförmigem Siliciumdioxid zudosiert, anschließend der pH-Wert auf 4 mit einer Na₂CO₃-Lösung bei pH 4 konstant gehalten.

Man erhält nach beiden Verfahren ein goldgelbes Pigment, das nach der üblichen Aufarbeitung bei 80 bis 130 °C getrocknet wird.

Um ein schwarzes Pigment zu erhalten, werden die mit FeO(OH) beschichteten Siliciumdioxid-Kugeln bei 300 bis 1000 °C im Wasserstoffstrom oder Formiergas (N₂/H₂ in verschiedenen Verhältnissen) erhitzt. Dabei wird schwarzes Eisen(II,III)-oxid erhalten.

Ein grünes Pigment wird erhalten, wenn die Siliciumdioxid-Kugeln mit Chromoxid beschichtet werden. Die Beschichtung erfolgt durch Zudosieren einer Chromchloridlösung zu einer auf 60 bis 90 °C erhitzten Suspension von Siliciumdioxid-Kugeln bei einem pH-Wert von 5,5 bis 9, wobei nach der üblichen Aufarbeitung das Pigment bei 500 bis 1000 °C getempert wird. Das erhaltene Pigment ist grün.

Durch Auffällen von Eisen(III)-hexacyaonoferrat(II) wird ein tiefblaues Pigment erhalten. Dazu werden eine wäßrige Eisen(III)sulfatlösung und eine wäßrige Kaliumhexacyanoferrat(II)lösung bei pH 1,8 bis 4 gleichzeitig zu einer auf 60 bis 90 °C erhitzten Dispersion des kugelförmigen Siliciumdioxids dosiert. Das Pigment wird auf übliche Weise aufgearbeitet und bei 80 bis 130 °C getrocknet.

Um eine gute Haftung der aus Berliner Blau bestehenden Beschichtung auf dem kugelförmigen Siliciumdioxid zu erreichen, ist es zweckmäßig, vorher zwischen 5 und 20 Gew.-% Titandioxid zur Aktivierung der Oberfläche auf das Siliciumdioxid aufzufällen.

Die Beschichtung mit Titandioxid erfolgt nach dem in US 3 553 001 und EP 0 803 550 beschriebenen Verfahren. Dabei wird zu der auf 50 bis 100 °C, insbesondere 70 bis 80 °C, erhitzten Suspension des kugelförmigen Siliciumdioxids langsam eine wäßrige Titansalzlösung zugegeben und es wird durch gleichzeitiges Zudosieren einer Base, wie z.B. wäßrige Ammoniaklösung oder eine wäßrige Alkalilauge, ein weitgehend konstanter pH-Wert von etwa 0,5 bis 5, insbesondere etwa 1,5 bis 2,5 eingehalten. Sobald die gewünschte Schichtdicke der TiO₂-Fällung erreicht ist, wird die Zugabe der Titansalzlösung gestoppt.

Dieses, auch als Titrationsverfahren bezeichnete Verfahren zeichnet sich dadurch aus, daß ein Überschuß an Titansalz vermieden wird. Das wird dadurch erreicht, daß man pro Zeiteinheit nur eine solche Menge der Hydrolyse zuführt, wie sie für eine gleichmäßige Beschichtung mit dem hydratisierten TiO₂ erforderlich ist und wie pro Zeiteinheit von der verfügbaren Oberfläche der zu beschichtenden Teilchen aufgenommen werden kann. Es entstehen deshalb keine hydratisierten Titandioxidteilchen, die nicht auf der zu beschichtenden Oberfläche niedergeschlagen sind. Die pro Minute zugesetzte Titansalzmenge liegt dabei in der Größenordnung von etwa 0,01 bis 2·10⁻⁴ Mol Titansalz pro Quadratmeter zu belegender Oberfläche.

Auch die allein mit Titandioxid beschichteten Siliciumdioxid-Kugeln sind als Absorptionspigment einsetzbar.

Der Metalloxidgehalt der beschichteten Siliciumdioxid-Kugeln beträgt 1 bis 80 Gew.-%.

Die erfindungsgemäßen Absorptionspigmente werden für die Pigmentierung von Lacken, Druckfarben und Kunststoffen verwendet, insbesondere werden sie aber in Formulierungen der pflegenden und dekorativen Kosmetik eingesetzt. Darunter sind Lippenstifte, Lidschatten, Nagellacke, flüssige und feste Makeups, Rouges, alle Arten von Puder, Emulsionen, Lotionen, Cremes, Lichtschutzpräparate und Formulierungen auf Fettbasis zu verstehen.

Die Pigmente werden in diese Formulierungen in einer Konzentration von 0,1 bis 80 Gew.-% eingearbeitet.

Die erfindungsgemäßen Pigmente weisen gegenüber konventionellen anorganischen Absorptionspigmenten eine Reihe von Vorteilen auf. Die Pigmente können in Öl- oder Wasserphasen durch Rühren eingearbeitet werden. Eine vorherige Mahlung ist nicht erforderlich. Die Pigmente entfalten sofort ihre volle Farbkraft, unabhängig von etwaiger Vorbehandlung. Dadurch wird die gewünschte Farbe einer Formulierung reproduzierbar und muß nicht ― wie bei gewöhnlichen Oxidpigmenten ― nach dem Vermahlen nochmals geprüft und eventuell eingestellt werden. Da die Farbe von Metalloxidpigmenten hauptsächlich von der Größe und Verteilung der Partikel bestimmt wird, wird durch die quasi-monodisperse Abscheidung der Metalloxidpartikel auf den Siliciumdioxid-Kugeln eine sehr gute Farbreinheit und Farbhomogenität erzielt.

Das Hautgefühl der erfindungsgemäßen Pigmente ist auf Grund der Kugelform und Kugelgröße ausgezeichnet. Während sich konventionelle Metalloxidpigmente rauh und stumpf anfühlen, sind die erfindungsgemäßen Metalloxide sehr weich und samtig.

Im Vergleich zu konventionellen Metalloxidpigmenten zeigen die erfindungsgemäßen Pigmente beim Benetzen eine deutlich geringere Farbänderung.

Mischungen aus Siliciumdioxid-Kugeln und konventionellen Metalloxidpigmenten zeigen in Anwendungssystemen wesentlich schwächere und weniger brillante Farben im Vergleich zu den erfindungsgemäßen Pigmenten.

**Tabelle 1**

| Farbvergleich zwischen erfindungsgemäßen Pigmenten und Mischungen aus Siliciumdioxid-Kugeln und Farbpigmenten **Rot** | | | | | |
|---|---|---|---|---|---|
| **Pigment** | **L-Wert** | **a-Wert** | **b-Wert** | **h°** | **C** |
| 50 % SiO₂-Kugeln + 50 % Fe₂O₃ | 47,95 | 24,01 | 16,36 | 34,2 | 29,05 |
| erfindungsgem. Pigment (SiO₂-Kugeln beschichtet mit Fe₂O₃) | 44,70 | 25,79 | 18,12 | 35,1 | 31,51 |

| **Gelb** | | | | | |
|---|---|---|---|---|---|
| **Pigment** | **L-Wert** | **a-Wert** | **b-Wert** | **h°** | **C** |
| 50 % SiO₂-Kugeln + 50 % FeO(OH) | 69,99 | 7,60 | 41,44 | 79,7 | 43,13 |
| erfindungsgem. Pigment (SiO₂-Kugeln beschichtet mit FeO(OH)) | 65,58 | 13,10 | 46,99 | 74,5 | 48,78 |

| **Weiß** | | | | | |
|---|---|---|---|---|---|
| **Pigment** | **L-Wert** | **a-Wert** | **b-Wert** | **h°** | **C** |
| 50 % SiO₂-Kugeln + 50 % TiO₂ | 92,68 | -0,60 | 0,47 | 142,0 | 0,76 |
| erfindungsgem. Pigment (SiO₂-Kugeln beschichtet mit TiO₂) | 96,24 | -0,50 | 1,74 | 105,9 | 1,81 |

| **Schwarz** | | | | | |
|---|---|---|---|---|---|
| **Pigment** | **L-Wert** | **a-Wert** | **b-Wert** | **h°** | **C** |
| 50 % SiO₂-Kugeln + 50 % Fe₃O₄ | 35,76 | 0,78 | -0,32 | 337,8 | 0,84 |
| erfindungsgem. Pigment (SiO₂-Kugeln beschichtet mit Fe₃O₄) | 33,41 | 0,40 | 1,47 | 74,9 | 1,52 |

| | | | | | |
|---|---|---|---|---|---|
| h°: Bunttonwinkel | | | | | |
| C: Farbstärke | | | | | |

Der Farbvergleich zeigt, daß die erfindungsgemäßen Pigmente eine deutlich höhere Farbstärke gegenüber den Mischungen aus Siliciumdioxid-Kugeln und konventionellen Metalloxidpigmenten besitzen. Weiterhin wird aus den L-Werten für Weiß und Schwarz deutlich, daß das erfindungsgemäße Weißpigment heller ist (höherer L-Wert) und das erfindungsgemäße Schwarzpigment dunkler ist (niedrigerer L-Wert). Das bedeutet, daß für das Erreichen des gleichen Effektes im Anwendungssystem von dem erfindungsgemäßen Pigment weniger eingesetzt werden muß.

Die nachfolgenden Beispiele sollen die Erfindung näher erläutern, ohne sie zu begrenzen.

### Beispiel 1

100 g Siliciumdioxid-Kugeln (Ronasphere®, Hersteller: Merck KGaA) werden in 1900 g vollentsalztem Wasser unter Rühren dispergiert, auf 75 °C erhitzt und mit 10%iger Salzsäure der pH-Wert auf 2,7 eingestellt. Anschließend wird eine 15%ige Eisen(III)chloridlösung zudosiert und durch gleichzeitige Zugabe von 32%iger Natronlauge der pH-Wert konstant gehalten. Die Dosiergeschwindigkeit der Eisen(III)chloridlösung beträgt in den ersten 20 Minuten 0,7 ml/min, für weitere 20 Minuten 2,4 ml/min und nach 40 Minuten 2,4 ml/min bis ein Bunttonwinkel von 45° erreicht ist. Der Bunttonwinkel wird mit dem Minolta Farbmeßgerät CR-300 gemessen. Die Suspension wird dann noch 30 min gerührt und danach der pH-Wert mit 32%iger Natronlauge auf pH 5 eingestellt. Der Feststoff wird über eine Nutsche abgetrennt, mit vollentsalztem Wasser salzfrei gewaschen, bei 110 °C getrocknet und für 30 min bei 800 °C geglüht. Man erhält ein rostrotes Pulver mit einem Gehalt an Eisen(III)oxid von 55 %.

### Beispiel 2

120 g Siliciumdioxid-Kugeln werden in 2277 g vollentsalztem Wasser unter Rühren dispergiert, auf 80 °C erhitzt und mit 20%iger Schwefelsäure der pH-Wert auf 3,2 eingestellt. Dann wird eine wäßrige Lösung von 338 g Eisen(III)sulfat und 46,4 g Eisen(II)sulfat Heptahydrat in 1000 g Wasser zudosiert. Durch gleichzeitige Zugabe von 32%iger Natronlauge wird der pH-Wert konstant gehalten. Die Dosiergeschwindigkeit beträgt in den ersten 10 min 0,6 ml/min, für weitere 10 min 1,3 ml/min und anschließend für weitere 10 min 1,9 ml/min. Nach 30 min wird die Dosiergeschwindigkeit auf 2,6 ml/min erhöht und die Dosierung der Eisensulfatlösung so lange fortgesetzt, bis ein Bunttonwinkel von 65° gemessen wird. Der pH-Wert wird dann mit 32%iger Natronlauge auf 7,0 eingestellt. Das Pigment wird dann wie in Beispiel 1 beschrieben, aufgearbeitet und bei 110 °C getrocknet. Man erhält ein goldgelbes Pulver mit einem Eisenoxidgehalt von 50 Gew.-%.

### Beispiel 3

10 g des nach Beispiel 2 hergestellten gelben Pigmentes werden in ein Quarzglasschiffchen gefüllt und in ein Quarzrohr überführt. Das Rohr wird 15 min mit Formiergas (Wasserstoff) gespült und für 30 min in einen auf 550 °C vorgeheizten Ofen überführt. Dann wird es aus dem Ofen genommen und unter weiterer Begasung mit Formiergas abkühlen gelassen. Das erhaltene Pigment ist schwarz. Das Pigment enthält 50 % Eisen(II,III)oxid (Fe₃O₄).

### Beispiel 4

50 g Siliciumdioxid-Kugeln werden in 950 g vollentsalztem Wasser unter Rühren dispergiert, auf 75 °C erhitzt und der pH-Wert auf 6,0 eingestellt. Anschließend wird eine wäßrige Lösung von 303,8 g Chromchlorid Hexahydrat in 777,8 g vollentsalztem Wasser zudosiert, wobei durch gleichzeitige Zugabe von 32%iger Natronlauge der pH-Wert konstant gehalten wird. Die Dosiergeschwindigkeit beträgt in den ersten 30 Minuten 1,25 ml/min. Sie wird anschließend auf 2,5 ml/min erhöht, bis die Chromchloridlösung zudosiert ist. Anschließend wird noch 30 min gerührt. Das Pigment wird dann, wie in Beispiel 1 beschrieben, aufgearbeitet und bei 110 °C getrocknet und für 30 min bei 800 °C geglüht. Man erhält ein dunkelgrünes Pulver mit Blaustich. Der Chromoxidgehalt beträgt 63,6 %.

### Beispiel 5

120 g Siliciumdioxid-Kugeln werden in 2280 g vollentsalztem Wasser unter Rühren dispergiert, auf 75 °C erhitzt und mit 10%iger Salzsäure der pH-Wert auf 2,0 eingestellt. Anschließend wird ein Gemisch aus 94,9 g 60%iger Titantetrachloridlösung und 83,2 g Wasser zudosiert und durch gleichzeitige Zugabe von 32%iger Natronlauge der pH-Wert konstant gehalten. Die Dosiergeschwindigkeit beträgt in den ersten 20 Minuten 1,0 ml/min und anschließend 2,2 ml/min. Nach beendeter Zugabe der Titantetrachloridlösung wird noch 30 min gerührt. Dann werden eine Lösung aus 57,5 g Eisen(III)sulfat in 575 g Wasser und eine Lösung von 74,9 g Kaliumhexacyanoferrat(II) in 575 g Wasser gleichzeitig aber getrennt zugegeben und der pH-Wert durch gleichzeitige Zugabe von 20%iger Schwefelsäure konstant gehalten. Die Dosiergeschwindigkeiten betragen in den ersten 20 Minuten 0,5 ml/min, für weitere 20 Minuten 1,0 ml/min und nach 40 min 2,0 ml/min. Nach Zugabe der beiden Lösungen wird noch 30 min gerührt. Das Pigment wird, wie in Beispiel 1 beschrieben, aufgearbeitet und bei 110 °C getrocknet. Man erhält ein blaues Pulver mit einem Gehalt an Berliner Blau von 37 Gew.-%.

### Beispiel 6

90 g Siliciumdioxid-Kugeln werden in 1710 g vollentsalztem Wasser unter Rühren dispergiert, die Suspension auf 75 °C erhitzt und mit 10%iger Salzsäure auf pH 2,2 eingestellt. Dazu wird eine Lösung, hergestellt aus 520,7 g einer 61,8%igen Titantetrachloridlösung und 552 g Wasser, zudosiert und durch gleichzeitige Zugabe von 32%iger Natronlauge der pH-Wert konstant gehalten. Die Dosiergeschwindigkeit beträgt in den ersten 60 Minuten 1,2 ml/min und anschließend 2,6 ml/min. Nach Zugabe der Titantetrachloridlösung wird noch 30 min gerührt und anschließend der pH-Wert mit 10%iger Salzsäure auf 7,0 eingestellt. Dann werden eine Lösung von 9,39 g Zinkchlorid in 220 g Wasser und eine Natriumsilikatlösung in 220 g Wasser und eine Natriumsilikatlösung, hergestellt aus 90,3 Natronwasserglas (27 Gew.-% SiO₂) und 233 g Wasser, gleichzeitig aber getrennt mit einer Geschwindigkeit von 2,5 ml/min zudosiert. Anschließend wird noch 30 min gerührt. Das Pigment wird, wie in Beispiel 1 beschrieben, aufgearbeitet, bei 110 °C getrocknet und bei 600 °C für 30 min geglüht. Man erhält ein weißes Pulver, bestehend aus 35,5 Gew.-% Siliciumdioxid-Kugeln, 53 Gew.-% Titandioxid, 2,0 Gew.-% Zinkoxid und 9,5 Gew.-% Siliciumdioxid.

### Beispiel 7

### Getönte Tagescreme

| | | |
|---|---|---|
| **A** | **SAP White** (SiO₂-Kugeln, beschichtet mit TiO₂, ZnO, SiO₂) | **4,00 %** |
| | **SAP Yellow** (SiO₂-Kugeln, beschichtet mit FeO(OH)) | **2,00 %** |
| | **SAP Orange** (SiO₂-Kugeln, beschichtet mit Fe₂O₃) | **0,20 %** |
| | **SAP Red** (SiO₂-Kugeln, beschichtet mit Fe₂O₃) | **0,20 %** |
| | **SAP Black** (SiO₂-Kugeln, beschichtet mit Fe₃O₄) | **0,20 %** |
| | Karion F flüssig (Sorbitol) | 5,00 % |
| | Allantoin | 0,50 % |
| | Keltrol T (Xanthan Gum) | 0,20 % |
| | Chemag 2000 (Imidazolidinyl Urea) | 0,30 % |
| | Euxyl K 400 (Methylbromo Glutaronitrile, Phenoxyethanol) | 0,10 % |
| | Methyl-4-hydroxybenzoat (Methylparaben) | 0,15 % |
| | Wasser, demineralisiert | ad 100,00 % |
| **B** | Arlacel 165 (Glyceryl Stearate, PEG-100 Stearate) | 2,50 % |
| | Montanov 68 (Cetearyl Alcohol, Cetearyl Glucoside) | 2,50 % |
| | Dow Corning 345 (Cyclomethicone) | 1,00 % |
| | Cetiol SN (Cetearyl Isononanoate) | 5,00 % |
| | Eutanol G (Octyldodecanol) | 4,00 % |
| | Sheabutter (Butyrospermum Parkii) | 4,00 % |
| | Propyl-4-hydroxybenzoat (Propylparaben) | 0,05 % |
| **C** | Parfümöl Bouquet Poudree | 0,10 % |

### Herstellung:

Alle Bestandteile der Phase A, außer Keltrol, im Wasser dispergieren. Das Keltrol unter Rühren einstreuen und nach 15 min auf 80 °C erhitzen. Phase B auf 75 °C erhitzen. Langsam Phase A in Phase B einrühren, homogenisieren. Bei 40 °C Phase C zugeben und unter Rühren abkühlen.

### Beispiel 8

### Lippenstift

| | | |
|---|---|---|
| **A** | **SAP Red** (SiO₂-Kugeln, beschichtet mit Fe₂O₃) | **10,00 %** |
| | **SAP White** (SiO₂-Kugeln, beschichtet mit TiO₂, ZnO, SiO₂) | **5,00 %** |
| **B** | Bienenwachs | 8,75 % |
| | Lunacera C 44 (Ceresin) | 5,25 % |
| | Adeps Lanae SP (Lanolin) | 3,50 % |
| | Isopropylmyristat | 5,60 % |
| | Paraffin dickflüssig (Paraffinum Liquidum) | 2,10 % |
| | Oxynex K flüssig (PEG-8, Tocopherol, Ascorbyl Palmitate, Ascorbinsäure, Zitronensäure) | 0,05 % |
| | Propyl-4-hydroxybenzoat (Propylparaben) | 0,10 % |
| | Rizinusöl | ad 100,00 % |
| **C** | Tendresse (Parfum) | 0,20 % |

### Herstellung:

Die Bestandteile der Phase B werden auf 75 °C erhitzt und aufgeschmolzen. Die Pigmente der Phase A werden zugegeben und alles gut durchrührt. Die Lippenstiftmasse wird dann in der auf 65 °C temperierten Gießapparatur 15 Minuten gerührt und parfümiert. Die homogene Schmelze wird in die auf 55 °C vorgewärmten Gießformen gegossen. Anschließend kühlt man die Formen ab und entfernt die Gießlinge kalt. Nach Erwärmen der Lippenstifte auf Raumtemperatur werden die Lippenstifte kurz abgeflammt.

### Beispiel 9

### Rouge

| | | |
|---|---|---|
| **A** | **SAP Red** (SiO₂-Kugeln, beschichtet mit Fe₂O₃) | **21,50 %** |
| | **SAP White** (SiO₂-Kugeln, beschichtet mit TiO₂, ZnO, SiO₂) | **7,00 %** |
| | **SAP Orange** (SiO₂-Kugeln, beschichtet mit Fe₂O₃) | **0,50 %** |
| | **SAP Yellow** (SiO₂-Kugeln, beschichtet mit FeO(OH)) | **0,50 %** |
| | **SAP Black** (SiO₂-Kugeln, beschichtet mit Fe₃O₄) | **0,50 %** |
| | Glimmer | 10,00 % |
| | Talkum | 18,00 % |
| | Weißer Ton (Kaolin) | 25,00 % |
| | Reisstärke | 5,00 % |
| | Magnesiumstearat | 2,00 % |
| **B** | Binder: | |
| | Isopropylmyristat | 8,00 % |
| | Dow Corning 1403 fluid (Dimethicone, Dimeticonol) | 1,00 % |
| | Dow Corning 200 (350 cs) fluid (Dimethicone) | 1,00 % |

### Herstellung:

Bestandteile der Phase A zusammengeben, vormischen und sieben (100 µm). Anschließend tropfenweise den Binder einrühren. Die Puder werden bei 40 bis 50 bar gepreßt.

### Beispiel 10

### Lidschatten

| | | |
|---|---|---|
| **A** | **SAP Blue** (SiO₂-Kugeln, beschichtet mit TiO₂ und Berliner Blau) | **10,00 %** |
| | **SAP Green** (SiO₂-Kugeln, beschichtet mit Cr₂O₃) | **7,00 %** |
| | **SAP White** (SiO₂-Kugeln, beschichtet mit TiO₂, ZnO, SiO₂) | **13,00 %** |
| | Glimmer | 10,00 % |
| | Talkum | 17,00 % |
| | Weißer Ton (Kaolin) | 24,50 % |
| | Reisstärke | 2,50 % |
| | Magnesiumstearat | 2,00 % |
| | Aerosil 200 (Silica) | 5,00 % |
| **B** | Binder: | |
| | Isopropylmyristat | 8,00 % |
| | Dow Corning Q2-1403 fluid (Dimethicone, Dimeticonol) | 1,00 % |
| | Dow Corning 200 (350 cs) fluid (Dimethicone) | 1,00 % |

### Herstellung:

Bestandteile der Phase A zusammengeben, vormischen und sieben (100 µm). Anschließend die Pudermischung unter Rühren tropfenweise mit dem Binder versetzen. Die Puder werden bei 40 bis 50 bar gepreßt.

## Patentansprüche

1. Anorganische sphärische Absorptionspigmente, dadurch gekennzeichnet, daß sie aus Siliciumdioxid-Kugeln mit einem Durchmesser von 50 nm bis 50 µm und einer Beschichtung aus einem Metalloxid mit Ausnahme von Titandioxid oder einem Metalloxid und einem farbgebenden Material bestehen.

2. Absorptionspigmente nach Anspruch 1, dadurch gekennzeichnet, daß das Metalloxid Fe₂O₃, FeO(OH), Fe₃O₄ oder Cr₂O₃ ist.

3. Absorptionspigmente nach Anspruch 1, dadurch gekennzeichnet, daß das farbgebende Material Berliner Blau, Carminrot, ein organisches Pigment oder ein organischer Farbstoff ist.

4. Verwendung der Absorptionspigmente nach den Ansprüchen 1 bis 3 zur Pigmentierung von Lacken, Druckfarben, Kunststoffen und Formulierungen der pflegenden und dekorativen Kosmetik.

5. Verwendung nach Anspruch 4, dadurch gekennzeichnet, daß die Formulierungen Lippenstifte, Lidschatten, Nagellacke, flüssige und feste Makeups, Rouges, Puder, Emulsionen, Lotionen, Cremes, Lichtschutzpräparate oder Formulierungen auf Fettbasis sind.
